# EUROPEAN PATENT APPLICATION

(11) **EP 1 510 231 A2**
(43) Date of publication of application: **02.03.2005**
(21) Application number: 04078146.0
(22) Date of filing: 30.09.2003
(51) Int. Cl.: A61M 11/06, A61G 10/00

(54) **Aerosol inhalation device and nebulizer**

(30) Priority: 30.09.2002 US 259644
(62) Divisional of application: 03021962.0
(71) Applicant: Baby's Breath Ltd., M.P. Hefer 38100 (IL)
(72) Inventor: Halamish, Asaf, Perdes Chana Karkur (IL)
(74) Representative: White, Duncan Rohan

(57) **Abstract**

The invention relates to a pneumatic nebulizer comprising at least one downwardly projecting aerosol flow outlet passageway; at least one inlet for introduction of a flow of high velocity gas; at least one orifice through which said flow of high velocity gas passes thereby causing a venturi effect; at least one source of liquid in fluid communication with said at least one orifice such that liquid is drawn to said orifice by said venturi effect, said liquid thereby forming into drops and flowing with said high velocity gas; and at least one baffle upon which said flow of high velocity gas and drops are impinged so as to atomize said drops into yet smaller droplets thereby forming a mist, said baffle deployed such that a flow-path of said mist substantially circumscribes said baffle so as to reach said at least one downwardly projecting aerosol flow outlet passageway.

## Description

### Field of the invention

The present invention relates in a first aspect to an aerosol inhalation device and in particular, it concerns a device for the delivery of a flow of aerosol medication to a breathing patient in a reclining position, with the device suspended above the patient's nose and mouth.

In a second aspect the present invention relates to pneumatic nebulizers and, in particular, it concerns a pneumatic nebulizer in which aerosol mist is produced in a downwardly flowing direction, the aerosol mist then leaves the nebulizer through a downwardly projecting aerosol outlet.

### Background art

In the patent application WO 02/02052, to the present inventor, discloses an aerosol inhalation interface that is suspended above the patient and that is particularly suitable for inhalation therapies requiring special breathing techniques, such as breathing in synchronization with the inhalation device or taking hard deep breath, these techniques being impossible for some patients such as infants and very young children, patients with limited mental capacity, patients who are very weak, or patients with limited lung capacity.

The device disclosed in WO 02/02052 is also particularly suitable for administering aerosol medications to infants and small children.

Said device comprises an aerosol generator, a housing, a downwardly projecting outlet sleeve and an air movement deflector; the air movement deflector, for example a hood-like object, defines a volume with a reduced ambient air movement in which the head of a supine patient can be set; a medicated aerosol is produced by the aerosol generator, is discharged in a containment region defined by the housing and is channeled downwards through the outlet sleeve in the volume with a reduced ambient air movement, where the patient can inhale it.

Providing an aerosol generator with good performances when used on an aerosol device like the one disclosed in WO 02/02052 involves considerable technical problems: at present it is known to provide a nebulizer for the production of a high volume of aerosol for inhalant delivery of medications. In general pneumatic nebulizers atomize liquids by introducing small amounts of the liquid into a flow of high velocity air, which serves to break up the liquid into small droplets. Usually, the liquid is as drawn from a reservoir and aspirated into the airflow by means of a venturi effect created by structural elements of the device. The air and liquid mixture then continues in an upward direction to an aerosol outlet. The medicated aerosol mist is then administered to a patient by means of a therapeutic inhalation interface or by discharge of the mist into the ambient air for direct inhalation by the patient. This upward flow has become the generally accepted means for keeping drops that are too large from continuing with the upward airflow. The larger drops are then drawn by gravity back into the reservoir. This style of nebulizer is herein referred to as an "up draft" nebulizer.

Some pneumatic nebulizers, such as the up draft nebulizer of U.S. Patent No. 6,338,443, add an additional step of impacting the high velocity air and liquid mixture onto a solid surface so as to further break up the liquid into smaller droplets, which are then drawn out through the aerosol outlet. Here too, drop which are too large fall back into the reservoir region from the force of gravity.

The nebulizer disclosed in U.S. Patent 5,490,630 has a side directed aerosol outlet, herein referred to as a "side draft" nebulizer, and includes an atomization stage in which a liquid is aspirated into a downward flowing jet of air. The air/liquid mixture in then impinged upon a solid structural surface to further atomize the droplets as the flow path continues in a downward direction. The air/liquid mixture is then directed upwardly toward the side directed aerosol outlet. The change of direction is used in this device as a means of separating out the larger drops from the aerosol mist.

It is obvious by their designs that the above mentioned devices are intended for deployment at a designated orientation, and that proper function is 1 united to a relatively small range of variance from the designated orientation. In practical use, however, it would sometimes advantageous to deploy a nebulizer at an orientation outside the tolerances of these devices. U.S. Patent 4,512,341 to Lester suggests a solution by disclosing a nebulizer that is operational at any orientation between vertical and horizontal. The vertical orientation, however, is referring to an up draft configuration, therefore '341 discloses a nebulizer that is usable as both an up draft and side draft nebulizer, and any orientation in between.

Further, WO0202052, to the present inventor, discloses an aerosol inhalation interface that is suspended above the patient. It should be obvious to one ordinarily skilled in the art that while the inhalation interface of WO0202052 will function with excellent results using either up draft or side draft nebulizers, it would be preferable to use such an interface in concert with a "downdraft" nebulizer. There are also numerous applications in which downwardly directed flow of medicated aerosol is preferable.

There are also known devices generally referred to as inhalers that are used by some patients, such as asthmatics, suffering respiratory distress. These devices typically consist of a pressurized vial from which a jet of medicated droplets is discharged in a downward direction into a side opening mouthpiece. These devices, however, are intended for delivery of medicated spray over a short time span, generally less than one second in duration. That is to say, a short burst of spray. Devices of this type are not structurally nor mechanically suited, nor are they intended for delivery for an extended time period of severally minutes, nor do they teach nor suggest such application.

There is therefore a need for a nebulizer having a downwardly facing aerosol outlet that is structurally configured so as to produce aerosol mist in a downward direction and that delivers a downwardly directed flow of medicated aerosol mist for a period of time. That is to say, a downdraft nebulizer.

### Summary of the invention

The object of a first aspect of the present invention is to improve the device disclosed with WO 02/02052, particulariy concerning the problem of adjusting the position of the outlet sleeve in front of the patient's mouth and nose zone.

This object of this first aspect of the present invention is accomplished with a device for delivering a flow of aerosol to a patient positioned supinely, comprising:
- an aerosol generator, suitable to generate an aerosol;
- a downwardly projecting outlet sleeve interconnected to said aerosol generator and comprising a bottom outlet suitable to be suspended above the patient's nose and mouth and being suitable to channel said aerosol and eject it through said bottom outlet in a region proximal to the mouth and nose of said patient;
- a cap housing said bottom outlet and suitable to house said patient's head, to reduce the ambient air movement in said region proximal to the mouth and nose of said patient under said cap and to inhibit the dispersion of said aerosol once it arrives at said region;
- adjusting means suitable to adjust the position of said bottom outlet within said region proximal to the mouth and nose of said patient under said cap.

The possibility of adjusting the position of said bottom outlet within said region proximal to the mouth and nose of said patient within said closed space allows and makes easy for a third person, for example a mother, to keep the bottom outlet closer to the patient's -e.g. a baby- mouth or nose, and to adjust it easier in case the patient moves his head: this allows to shape the bottom outlet so as to eject a concentrated aerosol jet and to direct it towards the patient's mouth or nose; the applicant found out that in this way of administering aerosol is more effective, and the patient assimilates a bigger amount of aerosol or medicament.

According to a further teaching of the present invention, the downwardly projecting outlet sleeve comprises, at its lower end a substantially non-divergent nozzle which defines said bottom outlet.

According to a further teaching of the present invention, said bottom outlet has a cross-section whose area is less than about 1300 mm² and that can be inscribed in a circle whose diameter is substantially smaller than 60 mm.

According to a further teaching of the present invention, said downwardly projecting outlet sleeve can comprise a bellows-like body through which said aerosol can be channelled, said bellows-like body being suitable to be deformed to adjust the position of said bottom outlet in said region proximal to the mouth and nose of the patient.

According to a further teaching of the present invention, said downwardly projecting outlet sleeve can comprise a telescopic tubular body through which said aerosol can be channelled.

According to a further teaching of the present invention, said cap defines one or more openings suitable to allow good oxygenation of the patient by means of sufficient change of air between the zone under said cap and the ambient out of said cap.

According to a further teaching of the present invention, said device for delivering a flow of aerosol can comprise at least one leg on which the device itself can rest.

According to a further teaching of the present invention, said device for delivering a flow of aerosol can comprise a connecting body supporting said sleeve, and said at least one leg can be connected to said connecting body through one or more first hinges in a way that said at least one leg can be folded.

According to a further teaching of the present invention, said at least one leg can comprise a plurality of segments connected one to another by means of one or more second hinges.

According to a further teaching of the present invention, said at least one leg can comprise a plurality of segments which can be assembled by fitting one in another.

According to a further teaching of the present invention, said at least one leg can be telescopic so that can be extended and retracted.

According to a further teaching of the present invention, said at least one leg can be reversibly connectable to and disconnectable from said connecting body.

According to a further teaching of the present invention, said cap can comprise a hood made of a flexible sheet-like material resting on said at least one leg when said device is in operative condition.

According to a further teaching of the present invention, said hood is disposable and is fastened to said at least one leg so as to be easily removable from said at least one leg.

According to a further teaching of the present invention, said device for delivering a flow of aerosol can further comprise objects hanging over the patient's head inside said air movement deflector, said objects being suitable to quiet, relax or amuse said patient.

The object of a second aspect of the present invention concerns a pneumatic nebulizer in which aerosol mist is produced in a downwardly flowing direction, the aerosol mist then leaves the nebulizer through a downwardly projecting aerosol outlet.

According to the teachings of this second aspect of the present invention there is provided a
pneumatic nebulizer comprising: a) at least one downwardly projecting aerosol flow outlet passageway; b) at least one inlet for introduction of a flow of high velocity gas; c) at least one orifice through which the flow of high velocity gas passes thereby causing a venturi effect; d) at least one source of liquid in fluid communication with the at least one orifice such that liquid is drawn to the orifice by the venturi effect, the liquid thereby forming into drops and flowing with the high velocity gas; and e) at least one baffle upon which the flow of high velocity gas and drops are impinged so as to atomize the drops into yet smaller droplets thereby forming a mist, the baffle deployed such that a flow-path of the mist substantially circumscribes the baffle so as to reach the at least one downwardly projecting aerosol flow outlet passageway.

According to a further teaching of the present invention, the high velocity gas is compressed air.

According to a further teaching of the present invention, the source of liquid is a reservoir configured as a bottom portion of a volume within a housing of the nebulizer.

According to a further teaching of the present invention, at least a top portion of the downwardly projecting aerosol outlet passageway extends upwardly into the volume.

According to a further teaching of the present invention, the volume substantially circumscribes at least a portion of the downwardly projecting aerosol outlet passageway.

According to a further teaching of the present invention, there is also provided at least one fluid passageway configured at a radial extremity of the volume through which the fluid communication is established. According to a further teaching of the present invention, there is also provided a fluid passageway insert deployed within the volume, the fluid passageway insert configured so as to substantially abut at least one housing surface defining the volume, the at least one fluid passageway configured as at least one surface groove configured in the surface of one of the fluid passageway insert and the housing surface.

According to a further teaching of the present invention., the volume is configured as a substantially vertical cylindrical volume such that the orifice is configured at a top of the cylindrical volume and the downwardly projecting aerosol outlet passageway is configured at a bottom of the cylindrical volume. According to a further teaching of the present invention, the fluid passageway insert is configured substantially as a cylinder having an open end and a closed end such that the closed end is deployed adjacent to the top of the cylindrical volume, and the closed end includes at least a portion of the orifice.

According to a further teaching of the present invention, the at least one surface groove is implemented as a plurality of circumferentially spaced apart grooves in the surface of one of the fluid passageway insert and the housing surface.

According to a further teaching of the present invention, the baffle is supported substantially above the downwardly projecting aerosol outlet passageway so as to constitute a flow path obstacle around which the mist flows in order to reach the downwardly projecting aerosol outlet passageway.

According to a further teaching of the present invention, the baffle is configured substantially as a disk having a diameter larger then a diameter of a top opening of the downwardly projecting aerosol outlet passageway, the baffle being supported above the downwardly projecting aerosol outlet passageway such that a extreme radial edge of the baffle extends beyond the top opening so as to be aligned with a portion of the reservoir.

According to a further teaching of the present invention, the baffle is supported by a plurality of circumferentially spaced apart support legs.

According to a further teaching of the present invention, the baffle further includes an upwardly extending protrusion, the baffle being deployed such that the protrusion is deployed substantially under the orifice and in substantially direct alignment with a flow of the high velocity gas and the drops so as to constitute a surface upon which the impingement occurs.

According to a further teaching of the present invention, a top surface of the baffle is downwardly sloping from the protrusion to the extreme radial edge, such that excess the liquid which accumulates on the baffle flows off the baffle thereby being returned to the reservoir.

According to a further teaching of the present invention, the aforesaid at least one source of liquid in fluid communication with said at least one orifice comprises:
- at least a first housing defining a concave bottom suitable to contain an amount of liquid;
at least a second housing defining an opening bounded by an edge of said second housing, said second housing being inserted inside said first housing, said edge of said second housing abutting against said concave bottom of said first housing so as to define an accumulation zone where an amount of liquid can accumulate, said accumulation zone being in fluid communication with said at least said one orifice through one or more notches or grooves made in said concave bottom and/or in said edge, such that said liquid can be drawn to said orifice by said Venturi effect, said liquid thereby forming into drops and flowing with said high velocity gas.

According to a further teaching of the present invention, the height of said one or more notches or grooves is lower than about 2 mm.

According to a further teaching of the present invention, said first housing and/or said second housing defines an annular groove surrounding said one or more notches or grooves made in said concave bottom and/or in said edge and collecting said liquid coming through said one or more notches or grooves from said accumulation zone.

According to a further teaching of the present invention, said at least one downwardly projecting aerosol flow outlet passageway defines an inner intake edge, said at least one baffle defines a concavity, said inner intake edge is substantially set inside said concavity, said baffle is deployed such that a flow-path of said mist substantially circumscribes said baffle so as to reach said inner intake edge of said at least one downwardly projecting aerosol flow outlet passageway.

There is also provided according a further teaching of the present invention, a pneumatic nebulizer comprising: a) at least one downwardly projecting aerosol flow outlet passageway; b) at least one inlet for Introduction of a flow of high velocity gas into the housing; c) at least one orifice configured in the housing through which the flow of high velocity gas passes thereby causing a venturi effect; d) a reservoir defined as a bottom region of volume within a housing of the nebulizer, the reservoir being in fluid communication with the at least one orifice such that liquid is drawn to the orifice by the venturi effect the liquid thereby forming into drops and flowing with the high velocity gas, a surface defining a bottom of the volume configured so as to be substantially planar, the plane of the bottom surface being at an angle so as to form a region of confluence of the liquid in the reservoir, the fluid communication being between the region of confluence and the at least one orifice; and e) at least one baffle upon which the flow of high velocity gas and drops is impinged so as to atomize the drops into yet smaller droplets thereby forming a mist, the baffle deployed such that a flow-path of the mist substantially circumscribes the baffle so as to reach the at least one downwardly projecting aerosol flow outlet passageway.

According to a further teaching of the present invention, the high velocity gas is compressed air.

According to a further teaching of the present invention, at least a top portion of the downwardly projecting aerosol outlet passageway extends upwardly into the volume.

According to a further teaching of the present invention the volume substantially circumscribes at least a portion of the downwardly projecting aerosol outlet passageway.

According to a further teaching of the present invention, there is also provided at least one fluid passageway configured at a radial extremity of the volume through which the fluid communication is established.

According to a further teaching of the present invention, there is also provided a fluid passageway insert deployed within the volume, the fluid passageway insert configured so as to substantially abut at least one housing surface defining the volume, the at least one fluid passageway configured as at least one surface groove configured in the surface of one of the fluid passageway insert and the housing surface.

According to a further teaching of the present invention, the volume is a substantially vertical cylindrical volume such that the orifice is configured at a top of the cylindrical volume and the downwardly projecting aerosol outlet passageway is configured at a bottom of the cylinder.

According to a further teaching of the present invention, the fluid passageway insert is configured substantially as a cylinder having an open end and a closed end such that the closed end is deployed adjacent to the top of the cylindrical volume, and the closed end includes at least a portion of the orifice, the bottom end configured with a radial bottom edge lying in a plane that is substantially parallel to the plane of the bottom surface of the volume.

According to a further teaching of the present invention, the baffle is supported substantially above the downwardly projecting aerosol outlet passageway so as to constitute a flow path obstacle around which the mist flows in order to reach the downwardly projecting aerosol outlet passageway.

According to a further teaching of the present invention, the baffle is configured substantially as a disk having a diameter larger then a diameter of a top opening of the downwardly projecting aerosol outlet passageway, the baffle being supported above the downwardly projecting aerosol outlet passageway such that a extreme radial edge of the baffle extends beyond the top opening so as to be aligned with a portion of the reservoir.

According to a further teaching of the present invention, the baffle is supported by a plurality of circumferentially spaced apart support legs. According to a further teaching of the present invention, the baffle further includes an upwardly extending protrusion, the baffle being deployed such that the protrusion is deployed substantially under the orifice and in substantially direct alignment with a flow of the high velocity gas and the drops so as to constitute a surface upon which the impingement occurs.

According to a further teaching of the present invention, a top surface of the baffle is downwardly sloping from the protrusion to the extreme radial edge, such that excess the liquid which accumulates on the baffle flows off the baffle thereby being returned to the reservoir.

There is also provided according to a further teaching of the present invention, a method for atomizing a liquid using a pneumatic nebulizer, the method comprising: a) providing a downward flow of high velocity gas; b) passing the downward flow of high velocity gas through at least one orifice, thereby causing a venturi effect; c) providing at least one source of liquid in fluid communication with the at least one orifice such that liquid is drawn to the orifice by the venturi effect, the liquid thereby forming into drops and flowing downwardly with the high velocity gas; and d) proving at least one baffle upon which the downward flow of high velocity gas and drops are impinged so as to atomize the drops into yet smaller droplets thereby forming a mist, the baffle deployed such that a flow-path of the mist substantially circumscribes the baffle so as to reach at least one downwardly projecting aerosol flow outlet passageway.

According to a further teaching of the present invention, the high velocity gas is implemented as compressed air.

According to a further teaching of the present invention, the source of liquid is implemented as a reservoir configured as a bottom portion of a volume within a housing of the nebulizer.

According to a further teaching of the present invention, the volume is implemented so as to substantially circumscribe at least a top portion of the downwardly projecting aerosol outlet passageway.

According to a further teaching of the present invention, there is also provided at least one fluid passageway configured at a radial extremity of the volume through which the fluid communication is established-According to a further teaching of the present invention, there is also provided a fluid passageway insert deployed within the volume, the fluid passageway insert configured so as to substantially abut at least one housing surface defining the volume, the at least one fluid passageway configured as at least one surface groove configured in the surface of one of the fluid passageway insert and the housing surface. According to a further teaching of the present invention the volume is implemented as a substantially vertical cylindrical volume such that the orifice is configured at a top of the cylindrical volume and the downwardly projecting aerosol outlet passageway is configured at a bottom of the cylindrical volume.

According to a further teaching of the present invention, the fluid passageway insert is implemented substantially as a cylinder having an open end and a closed end such that the closed end is deployed adjacent to the top of the cylindrical volume, and the closed end includes at least a portion of the orifice.

According to a further teaching of the present invention, the at least one surface groove is implemented as a plurality of circumferentially spaced apart grooves in the surface of one of the fluid passageway insert and the housing surface.

According to a further teaching of the present invention, the baffle is implemented so as to be supported substantially above the downwardly projecting aerosol outlet passageway so as to constitute a flow path obstacle around which the mist flows in order to reach tiie downwardly projecting aerosol outlet passageway.

According to a further teaching of the present invention, the baffle is implemented substantially as a disk having a diameter larger then a diameter of a top opening of the downwardly projecting aerosol outlet passageway, the baffle being supported above the downwardly projecting aerosol outlet passageway such that a extreme radial edge of the baffle extends beyond the top opening so as to be aligned with a portion of the reservoir.

According to a further teaching of the present invention., the baffle is implemented so as to be supported by a plurality of circumferentially spaced apart support legs.

According to a further teaching of the present invention, there is also provided an upwardly extending protrusion on a top surface of the baffle, the baffle being deployed such that the protrusion is deployed substantially under the orifice and in substantially direct alignment with a flow of the high velocity gas and the drops so as to constitute a surface upon which the impingement occurs.

According to a further teaching of the present invention, the baffle is implemented having a top surface that is downwardly sloping from the protrusion to the extreme radial edge, such that excess the liquid which
accumulates on the baffle flows off the baffle thereby being returned to the reservoir.

Further advantages obtainable with the present invention will be apparent upon consideration of the following description of some preferred and non limiting embodiments and accompanying drawings.

### List of Figures

Figure 1 is a first view in perspective of a preferred embodiment of a device for delivering a flow of aerosol medication according to the present invention;
Figure 2 is a side cross section view of the device of Figure 1;
Figure 3 is a second view in perspective of the device of Figure 1, showing the cap to reduce the ambient air movement in said region proximal to the mouth and nose of the patient;
Figure 4 is a perspective view of the joint inner body of the device of Figure 1;
Figure 5 is a side view, in cross-section, of the coupling between the joint inner body and the outlet sleeve of the device of Fig. 1;
FIG. 6 is a side perspective view of a first preferred embodiment of a downdraft nebulizer constructed and operative according to the teachings of the second aspect of the present invention;
FIG. 7 is a top elevation of the embodiment of Fig. 6, which serves to establish cross-sectional lines A-A and B-B:
FIG. 8 is a cross-sectional view of the embodiment of FIG. 6, along line A-A;
FIG. 9 is a cross-sectional view of the embodiment of FIG. 6, along line B-B;
FIG. 10 is a detail of region C of FIG. 8;
FIG. 11 is a cross-sectional view of an upper housing of the embodiment of FIG. 6 constructed and operative according to the teachings of the present invention, taken along line A-A;
FIG. 12 is a detail of region D of FIG. 11;
FIG. 13 is a cross-sectional view, taken along line A-A, of a fluid passageway insert constructed and operative according to the teachings of the present invention, for deployment in the upper housing of FIG. 11;
FIG. 14 is a detail of region E of FIG. 13;
FIG. 15 is a cross-sectional view, taken along line A-A, of a lower housing of the embodiment of FIG. 6 constructed and operative according to the teachings of the present invention;
FIG. 16 is a top elevation of an atomization baffle constructed and operative according to the teachings of the present invention, for deployment in the lower housing of FIG. 11, shown to establish cross-sectional line F-F;
FIG. 17 is a cross-sectional view, taken along line F-F, of the atomization baffle of FIG. 16;
FIG. 18 is a perspective view of a second preferred embodiment of a downdraft nebulizer constructed and operative according to the teachings of the second aspect of the present invention, this embodiment having a reservoir with a sloping bottom;
FIG. 19 is a side elevation of the embodiment of FIG 18; and
FIG. 20 is a cross-sectional view of the embodiment of FIG. 18.
Figure 21 shows a side view of a cross-section of a third preferred embodiment of a nebulizer according to the second aspect of the present invention;
Figure 22 shows a side view of a cross-section of the inner upper housing of the nebulizer of Figure 21;
Figure 23 shows a perspective view of the inner upper housing of Figure 22.

### Detailed description of the invention

The overall structure of the device for delivering a flow of aerosol medication

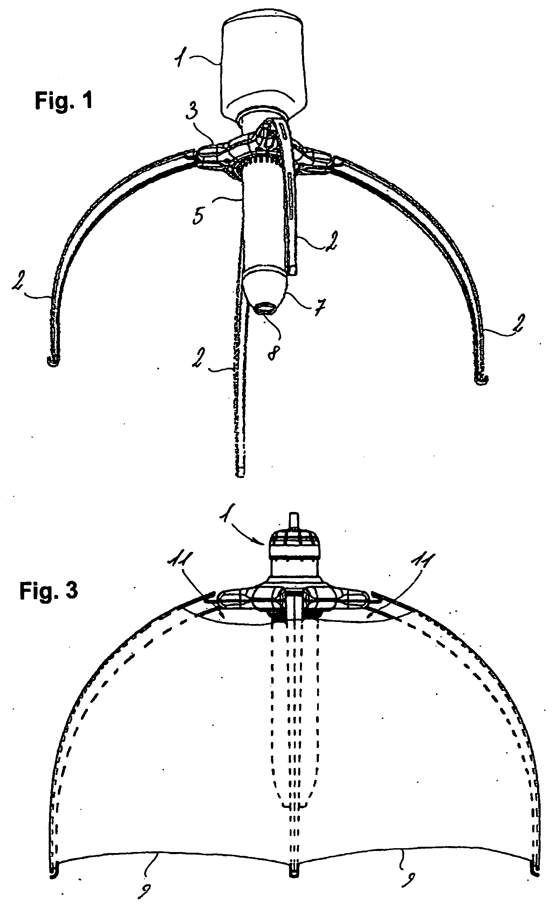

The preferred embodiment of a device for delivering a flow of aerosol medication according to the first aspect of the present invention and shown in Figures 1-3 comprises an aerosol generator 1 suitable to generate a downward aerosol flow; this aerosol generator can be, advantageously but not necessarily, the aerosol generator described in the international patent application WO 02/02052.

The aerosol generator 1 is fixed and supported by a lower structure comprising four legs 2 and a connecting body 3 to which the legs 2 are fixed, for example by means of hinges 4 (Figure 2; in the present specification hinges are also referred to as "first hinges 4") so that they can be folded when the structure must be put away -in this case, preferably the legs can be blocked in the opened position for example with a snap hook mechanism or a ball+spring block.

The connecting body 3 also defines a seat for housing a tubular body 5 which is part of an outlet sleeve -indicated with the overall reference numeral 6- and which projects downwards; on the lower end of the tubular body 5 is mounted a hollow tip 7 preferably made of soft material -such as soft polyethylene- or sylicone elastomer, thermoplastic elastomer (TPE) or other synthetic resins with suitable softness in order not to hurt or irritate the patient's head, in particular a baby's mouth or nose.

The hollow tip 7 defines an opening or bottom outlet 8.

The upper end of the tubular body is connected to the outlet of the aerosol generator 1 so as to channel downwards the aerosol produced by the aerosol generator 1 and eject it through the bottom outlet 8 in a region proximal to the mouth and nose of the patient.

The device for delivering a flow of aerosol medication shown in the appended Figures further comprises a cap 9 (Figure 3) configured to inhibit the dispersion of said aerosol once it arrives at the region proximal to the mouth and nose of the patient; the cap 9, in the embodiment shown, comprises a curtain or hood of a sheet-like material which, once the structure is laid on a floor, desk or analogous surface, defines a substantially closed space around the patient's head and hence reduces the ambient air movement in said closed space. However, preferably the hood of the cap is designed so as not to define a sealed closed space, but to allow a good air change with external air, in order to avoid stagnation of CO2 breathed out by the patient under the cap and not to give a suffocation feeling to the patient; in the embodiment of Figure 3, the cap is provided with suitable openings 11 or valves to ensure a good exchange of external air and a good oxygenation of the patient; alternatively, the cap doesn't reach the surface where the structure is put on and the bottom ends of legs 2, but ends above said surface in a way to define with the floor a proper passage for external air.

According to the present invention, the device for delivering a flow of aerosol medication further comprises adjusting means suitable to adjust the position of the bottom outlet 8 within the aforesaid region proximal to the patient's mouth and nose within said closed space defined by the cap 9.

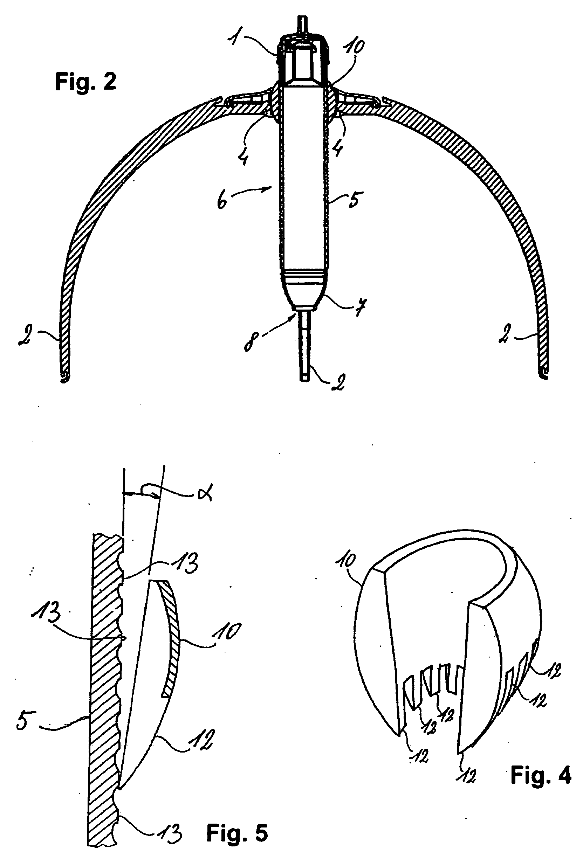

In the embodiment of Figures 1-5 the tubular body 5 is substantially one rigid body, that is not deformable along its longitudinal direction or normally to that direction in order to adjust the position in the space of the bottom outlet 8; the aforesaid seat -defined on the connecting body 3- for housing the tubular body 5 is an approximately cylindrical but slightly tapered through seat defined by a plurality of ribs 12 set to form a ring (Figures 4, 5; in Figure 5, angle α indicates the taper angle of the through seat defined by ribs 12); each rib 12 is a cantilever beam fixed to and protruding from a joint inner body 10. The upper external part of the joint inner body 10 defines a part of a substantially spherical surface and couples with a spherical cavity -that is a cavity defining a part of a spherical surface- defined in the connecting body 3 so as to form a ball-and-socket joint which allows to manually adjust the inclination in the 3-dimensional space of the tubular body 5.

In the embodiment shown in Figures 1-5, the external surface of the tubular body 3 is covered with a plurality of annular ribs 13 -Figure 5- having a rounded top edge; the annular ribs 13 engages -with a proper and relatively small interference- with the comers of the free ends of the cantilever ribs 12 of the joint inner body 10 (Figure 5), so that the tubular body 5 can be made sliding relatively to the joint inner body 10 gently pushing or pulling it with a hand, and the bottom outlet 8 can be displaced in the inner space under the cap 9.

In the embodiment shown in Figures 1-5 The aforesaid adjusting means comprises the annular ribs 13, the ball-and-socket joint to which the joint inner body 10 belongs and the cantilever ribs 12.

Preferably but not necessarily the downward projecting outlet sleeve 6 comprises, at its lower end, a substantially non-divergent nozzle, where the expression "non-divergent nozzle" in the present application means a nozzle whose cross-section decreases or remains substantially constant but doesn't become larger in the direction of flow; for example a divergent nozzle is not a non-divergent nozzle, a cylindrical outlet duct is a non-divergent nozzle.

Preferably the bottom outlet 8 has a cross section the area of which is not greater than about 1300 mm² , and the bottom outlet cross-section can be inscribed in a circle the diameter of which can be inscribed in a circle whose diameter is smaller than about 60 mm; for example the bottom outlet 8 of the described embodiment has a substantially circular cross-section with an area of 250-260 mm².

The substantially small and squat cross-section of the bottom outlet 8 as defined above, and the matter that the bottom outlet 8 is the opening of a non-divergent nozzle separately or in combination allow obtaining a substantially concentrated aerosol jet which can be directed towards the patient's mouth and/or nose; this way the patients breathes in a greater part of medicine than for example by spreading and saturating with aerosol the whole space under the cap 9, and the medicine is administered more efficiently.

In the embodiment shown in Figures 1-5 The aerosol generator 1 and eventually - depending on the position of tubular body 5- the upper end of the tubular body 5 protrudes over and outside the cap so that a third person ―such as, for example, the baby's mother- can grasp it like a handle and easily adjust from outside the position of the bottom outlet 8 inside the cap 9, placing the bottom outlet 8 and the concentrated aerosol jet in the best position for a proper inhalation and eventually following the movements of the baby's head, with no need to introduce a hand inside the cap 9 or somehow open it.

According to a preferred embodiment, the cap 9 comprises a cover of a sheet-like foldable or however flexible material which rests on the foldable legs 2 when they are opened in an operative condition, and said cover is disposable and arranged to be easily removable from the legs 2, to be thrown away or washed after use, for instance, the sheet-like material is hooked to suitable hooks made on the legs 2 or fixed with Velcro pads: these embodiment are particularly advantageous in hospitals, creches and analogous public facilities. The sheet like material can be any suitable material, for example a plastic transparent film, a wowen-non-wowen fabric, cellulose derivatives, a net with small holes etc.

In other applications -for example home applications- the sheet like material is not disposable, and can be fixed to the legs 2 in a way to be removable or not removable from the legs 2.

Using a transparent sheet-like cover has the advantage that the patient, specially a baby, during the aerosol session can see his mother or other known persons, and therefore it's easier to keep him or her quiet, and a quiet baby inhales a greater amount of aerosol than a crying baby, which expels aerosol more.

For similar purposes, advantageously the legs 2 or other inner parts of the cap 9 are provided with proper means for hanging objects which help in relaxing, diverting and keeping quiet a baby-patient, like for example hanging coloured objects, toys, puppets or windmills.

Moreover a transparent sheet-like cover allows the care person to see the baby and to make sure that the opening 8 is close to the mouth and to the nose of the infant.

The above described device is a non limiting embodiment susceptible of various modifications and variants without departing from the scope of the present invention: for example the height of the downwardly projecting outlet sleeve 6 over the patient's head can be adjusted by means of a telescopic tubular structure, or making one rigid tubular body slide inside a seat like the one defined by the connection body 3, and blocking the tubular body on it by means of a screwed ring, a screw, a screwed pin or a blocking spring; the downwardly projecting outlet sleeve 6 can comprise a bellows-like tubular body which can be deformed, bent, stretched or shrink it to adjust the position of the bottom outlet 8 inside the cap; the bottom outlet 8 can have also non-circular cross section, for example elliptic, elongated, square or polygonal cross-section; the ball-and-socket joint can be substituted with a Hooke's joint or other suitable joint allowing to adjust the inclination of the sleeve 5; the number of legs 2 can be different from one leg or four legs; the legs 2 can consist of a plurality of rigid separated segments which can be assembled by fitting or anyway connecting them together, or can consist of a plurality of rigid separated segments each of them is connected with a hinge to the end of another of these segments ―in the present specification also referred to as "second hinges"-, so that the folded legs can have a smaller overall length than when they are spread in operative condition; yet an aerosol device according to the present invention can be provided with telescopic legs which can be retracted when the aerosol device must be put away, and extended for operation; the legs 2 can be connected to the connecting body 3 by means of a fitting so that the legs can be disconnected when the aerosol device must be put away, and can be reconnected when the device must be used; the upper external part of the joint inner body 10 not necessarily defines a part of a substantially spherical surface but may also define another surface suitable to couple with the spherical cavity defined in the connecting body 3.

The second aspect of the present invention concerns a pneumatic nebulizer in which aerosol mist is produced in a downwardly flowing direction, the aerosol mist then leaves the nebulizer through a downwardly projecting aerosol outlet, that is to say, a downdraft nebulizer.

By way of introduction, the nebulizers presently in general use are configured for discharge of medication mist either into an inhalation interface or directly into the environment. Typically, the intention of nebulizers used for extended time administration is produce a flow of droplets of medication in atmospheric suspension to an inhalation interface, usually a mouth piece or a mask. The medicated mist is generally discharged in an upward or sideward direction since this is the easiest way to deal with the problem of trapping drops that are too large to be aspirated by a patient.

There are, however, applications where a downward flow may be advantageous and even preferred. An application in point is the aerosol inhalation interface that is suspended above the patient, disclosed in W002/02052., to the present inventor. An intention of WO02/02052 is to use the force of gravity to draw a medicated mist down to a patient lying beneath a nebulizer within a controlled environment.

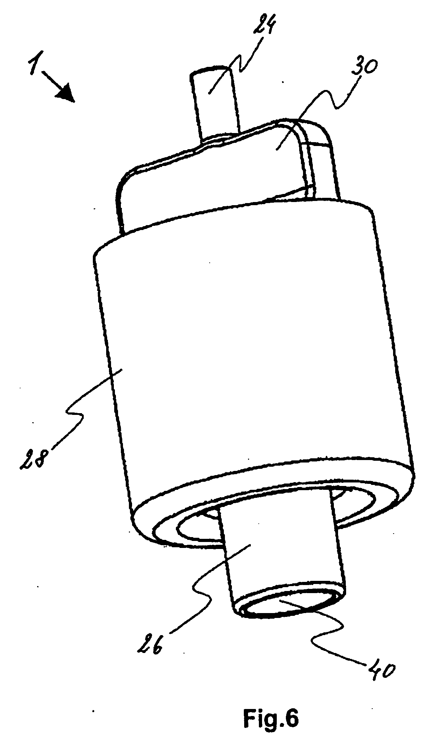

Referring now to the drawings, Figure 6 illustrates the exterior of a first preferred embodiment of a nebulizer, generally referred to herein by 1, constructed and operative according to the teachings of the present invention. High velocity gas, typically compressed air, is introduced into the nebulizer through the air inlet 24. medicated mist is discharged from the nebulizer through the downwardly projecting aerosol outlet passage 40 in the outlet tube 26, which extends downwardly from the lower housing 28. As used herein, the terms "down" and "downward", refer to a direction aligned with the force of gravity.

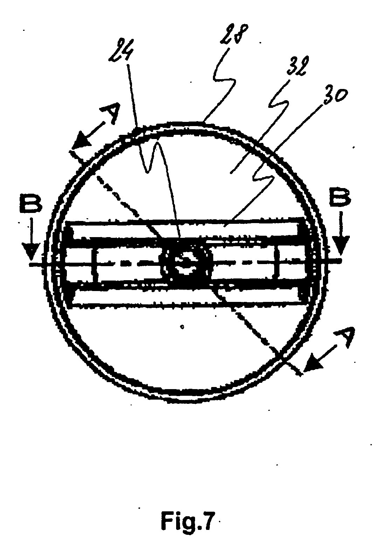

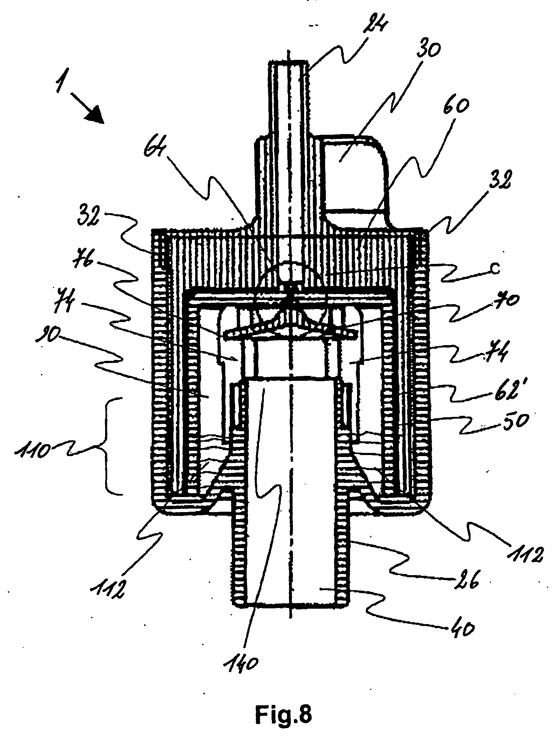

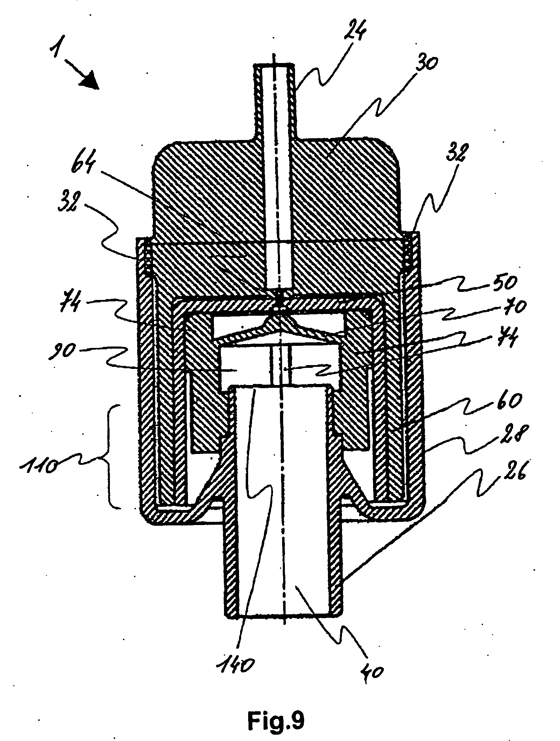

As mentioned above, Figure 7 serves to establish cross-sectional lines A-A, along which Figures 8, 11, 13, and 15 are taken, and B-B, along which Figure 9 is taken.

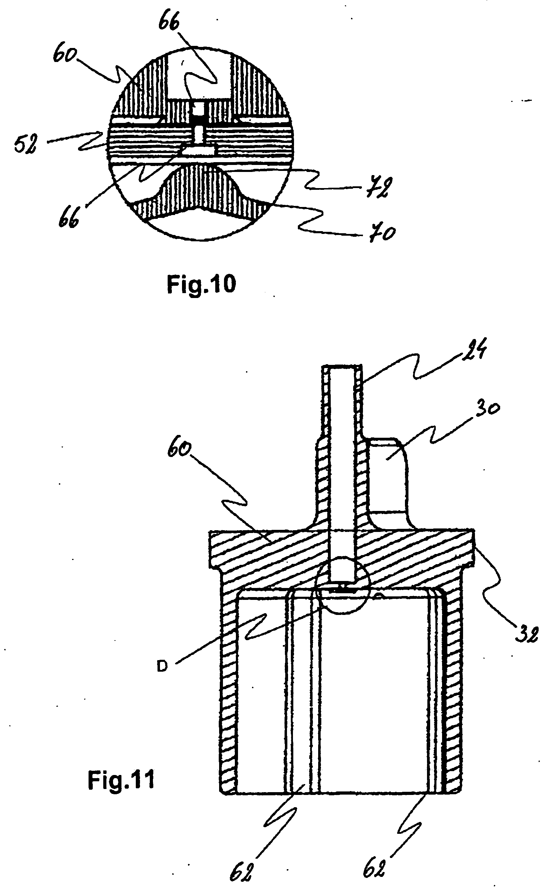

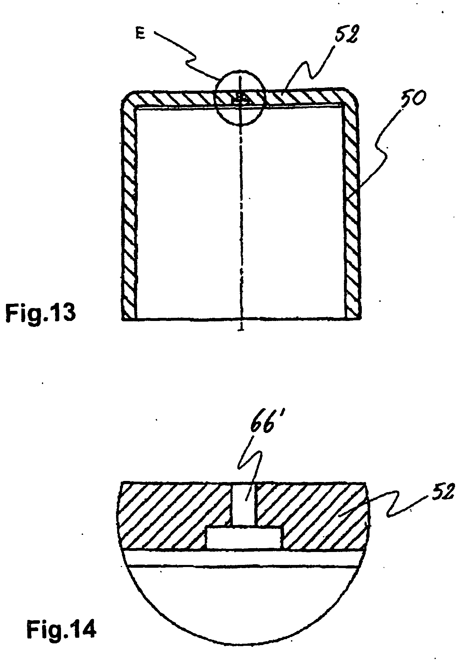

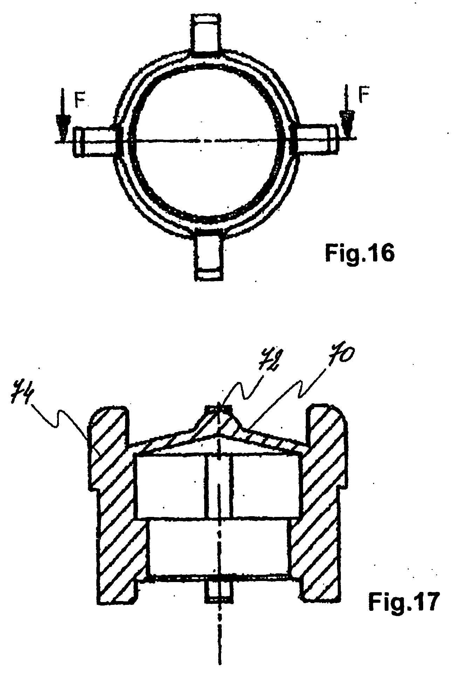

A first preferred embodiment, as discussed herein, includes four structural elements, the deployment of which is illustrated in Figures 8 and 9, which are cross-sections across the diameter of a substantially cylindrical housing taken along different lines (see Figure 7). A substantially cylindrical lower housing 28 includes a downwardly projecting aerosol outlet passageway 40 (for clarity see Figure 15). Supported above the opening of the aerosol outlet passageway 20 is an atomization baffle 70 (Figures 16 and 17). A substantially cylindrical upper housing 60 (Figure 11) is inserted into the lower housing 28. The inside diameter of the lower housing 28 and the outside diameter of the upper housing 60 as substantially the same so as to create a seal between them. Each of the two housing elements is configured with corresponding threads about their circumferences in region 32 so as to provide for secure interconnection of the two as the upper housing is turned using handle 30. The cylindrical wall of the upper housing extends substantially to the lower interior surface of the lower housing. When thusly interconnected, the two housing elements define within them an interior volume 90, the bottom of which serves as a reservoir 110 for the liquid 112 being dispensed by the nebulizer. Deployed within the volume 90 is a substantially cylindrical fluid passageway insert 50 (Figure 13) configured to substantially abut the cylindrical wall of the upper housing 60- The lower edge of the cylindrical wall of the fluid passageway insert 50 is spaced above the lower interior surface of the lower housing 28 enough to allow liquid 112 to flow beneath it.

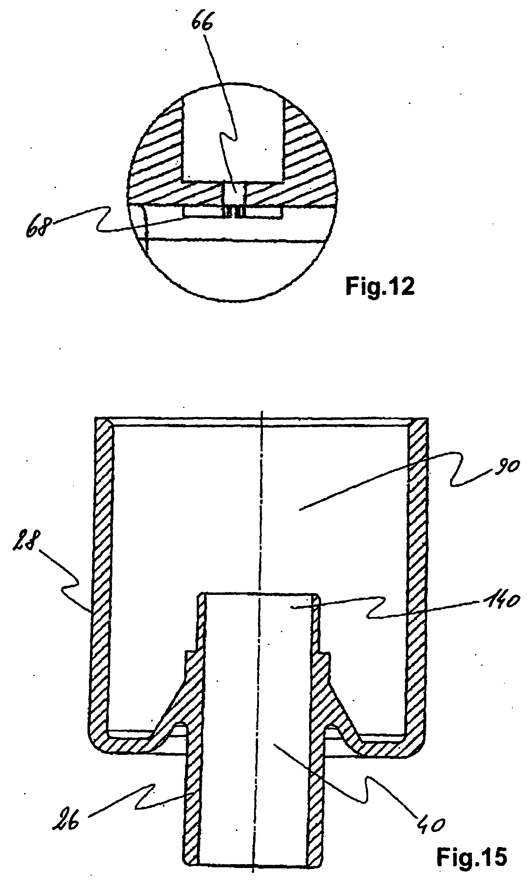

The upper housing 60 is configured with two structural features. One of these features is a terminal opening 66 (see Figure 12 for clarity) of the compressed air inlet 24. An outlet of the passageway 66, which is an upper portion of a venturi orifice 64, is circumscribed by a spacer extension 68 that serves to limit the height to which the fluid passageway insert 50 may be inserted into the upper housing 60. The space created between the fluid passageway insert 50 and the upper housing 40 serves as an upper portion of a fluid passageway 62. Another feature is spaced apart grooves 62 (see Figure 11) in the surface of the cylindrical wall that works in concert with the fluid passageway insert 50 to define vertical portions of fluid passageways 62. Thus, with the fluid passageway insert 50 deployed within the upper housing 60, fluid passages 62, through which fluid communication between the fluid reservoir region 110 of the interior volume 90 of the housing and the venturi orifice 64 may be established, are defined. Note that for graphic purposes, the fluid passages 62' are illustrated in black in Figure 8. The lower portion 66' of the venturi orifice 64 is configured in the upper wall 52 of the fluid passageway insert 50. It should be noted that the four grooves 62 that partially define the fluid passageways 62' may be implemented as any number of grooves.

Is should be further noted that the grooves 62 described here, and with regard to a second embodiment of the present invention discussed below, may alternately be configured in the fluid passageway insert 50.

Deployed with in the internal volume of the nebulizer, below the opening of the venturi orifice 64 is an atomization baffle 70. The atomization baffle 70 is configured substantially as a disk deployed at an orientation substantially perpendicular to the cylindrical wall defining the side of the internal volume. As illustrated here, and in Figures 9, 16 and 17, the atomization baffle 70 is supported by four radially spaced apart legs 74 that rest of the upper end 140 of the aerosol outlet tube 26. It should be noted that the baffle may alternatively be supported by connection to the fluid passageway insert. The upper surface of the atomization baffle 70 is configured with a semispherical protrusion 72 located substantially below and adjacent to the opening of the venturi orifice 64. It should be noted that the protrusion may be of any functionally appropriate shape, such as, but not limited to, conical, geodesic, or flat. The remainder of the upper surface of the atomization baffle 70 is downwardly sloping such that liquid that collects on the surface during the atomization process will flow to the extreme radial edge 76 of the atomization baffle 70 and fall back into the fluid reservoir region 110 of the internal volume 90.

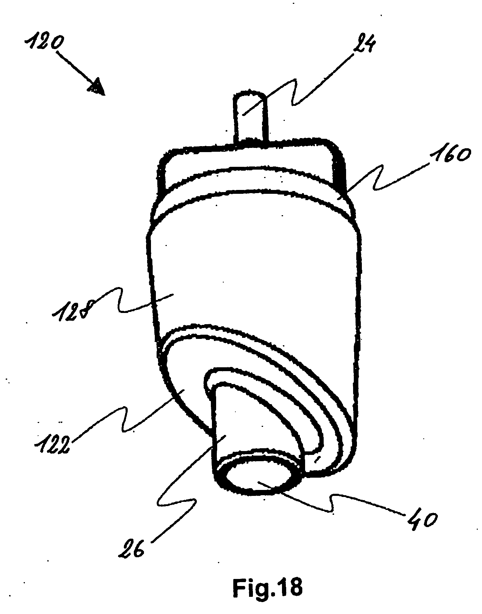

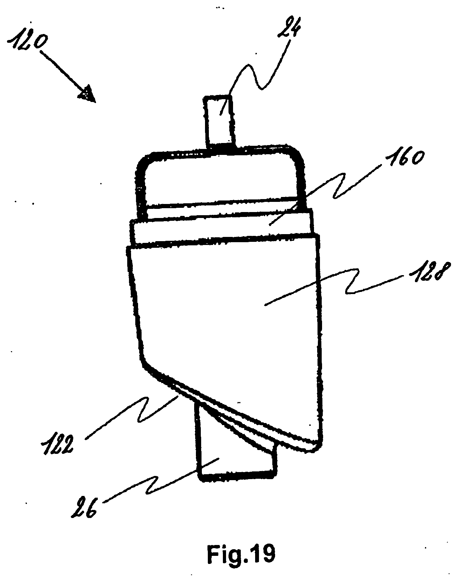

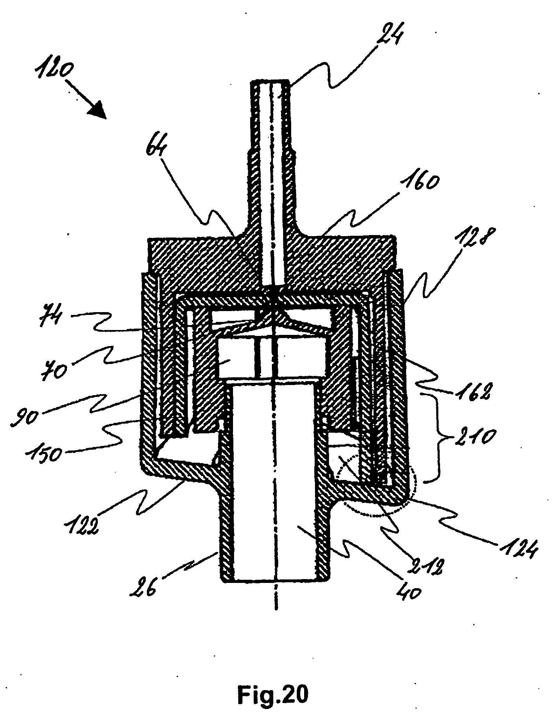

A second embodiment of a nebulizer, generally referred to as 120 constructed and operative according to the teachings of the present invention is illustrated in Figures 18-20. The operational principles of this second embodiment are the same as the first embodiment described above, therefore, structural elements effecting the operation are similar to the first embodiment and therefore are numbered accordingly.

The differentiating feature of this second embodiment is the sloping bottom 122 of the lower housing 128. The sloping bottom 122 creates a region of liquid confluence 124 in the liquid reservoir 210, This allows more of the liquid 212 to be aspirated by the fluid passageway 162 which is configured with a bottom opening substantially corresponding to the lowest point in the reservoir. That is, the inside surface of the cylindrical wall of the upper housing has a groove extending upward from an area adjacent to the lowest point to the top of the wall, and the outer surface of the cylindrical wall of the fluid passageway insert abuts substantially the rest of the inside surface of the cylindrical wall of the upper housing.

The sloping bottom also allows for proper operation of the nebulizer at varying orientations other than vertical in this embodiment the slope of the bottom is preferably 30 degrees from vertical and allows full operation of the nebulizer within a range of orientations between vertical and about 25 degrees from vertical.

In this second embodiment the lower extremities of each of the cylindrical walls of the upper housing 160 and fluid passageway insert 150 lie within planes that are substantially parallel to the plane of the sloping bottom 122 of the lower housing 128. The upper and lower housing elements may be secured by friction or pressure when press together.

The operation of a nebulizer constructed and operative according to the teachings of the present invention, is substantially as follows:
1 -Liquid to be atomized in placed in the reservoir region 110 of the lower housing 28, in which the atomization baffle 70 is deployed.
2 -The upper housing 60, containing the fluid passageway insert 50, is inserted into the lower housing 28 and secured by turning the upper housing 60 using handle 30.
3 -A tube connected to a source of compressed air is connected to inlet 24 and a high velocity flow of air is introduced into the nebulizer.
4 -As the high velocity air passes through the venturi orifice 64, liquid from the reservoir 110 is aspirated up through the fluid passageways 62', by a venturi effect, and into the air flow.
5 -The introduction of the liquid into the flow of high velocity air causes the liquid to form drops.
6 -As the drops flow out of the venturi orifice 64 they are impinged on the surface of the semispherical protrusion 72 of the atomization baffle 50. This causes some of the drops to break up into smaller droplets so as to form a mist.
7 -The mist follows a flow path that flows radially outward around the atomization baffle 70.
8 -The flow path of the mist turns radially inward toward the top 140 of the downwardly projecting aerosol outlet passageway 40.
9 -The mist flows down through the aerosol outlet passageway 40 and out of the nebulizer with a downward flow path.
10 -Liquid from drops that where to large to be suspended in the mist fall, by the force of gravity, onto the upper surface of the atomization baffle 70 or directly into the liquid reservoir 110. Any liquid that condenses on the upper surface of the atomization baffle 70 flows to the outer or extreme radial edge 76 of the baffle and fall into the liquid reservoir 110, Liquid that condenses on other surfaces within the interior volume of the nebulizer flows by the force of gravity back into the liquid reservoir 110.

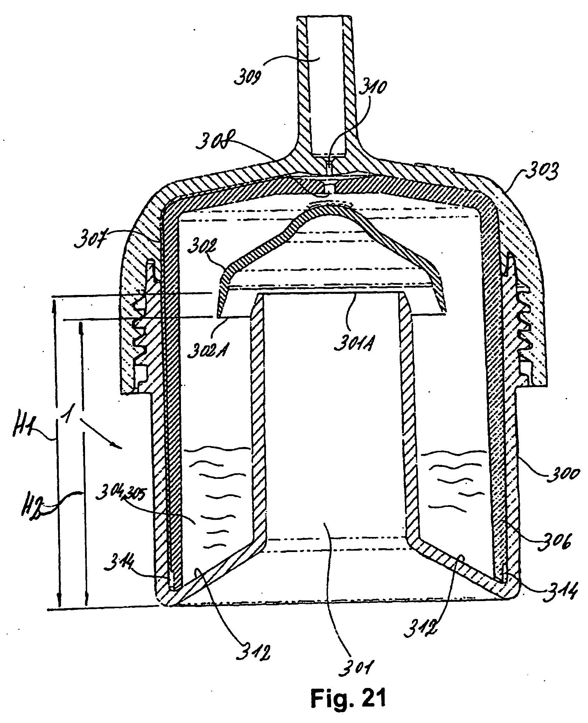

A third non limiting embodiment of a nebulizer, generally referred to as 1', constructed and operative according to the teachings of the second aspect of the present invention, is illustrated in Figures 21-23: this nebulizer has some improvements with respect to the embodiments shown in Figures 6-20.

The improved nebulizer 1 of Figures 21-23 comprises a substantially cylindrical lower housing 300 -in the present specification also referred to as "first housing 300"- defining a downwardly projecting aerosol outlet passageway 301 (Figure 21). Supported above the opening of the aerosol outlet passageway 301 is an atomization baffle 302. A substantially cylindrical outer upper housing 303 is inserted into the lower housing 300. The inside diameter of the lower housing 300 and the outside diameter of the outer upper housing 303 are substantially the same so as to create a seal between them.

The cylindrical wall of the outer upper housing 303 extends substantially to the lower interior surface of the lower housing. When thus interconnected, the two housings 300, 303 define within them an interior volume, the bottom of which serves as a reservoir 304 for the liquid 305 being dispensed by the nebulizer. A further housing 306 (Figures 21-23) -in the present specification also referred to as "second housing 306" or "inner upper housing 306"- is inserted inside the housings 300, 303.

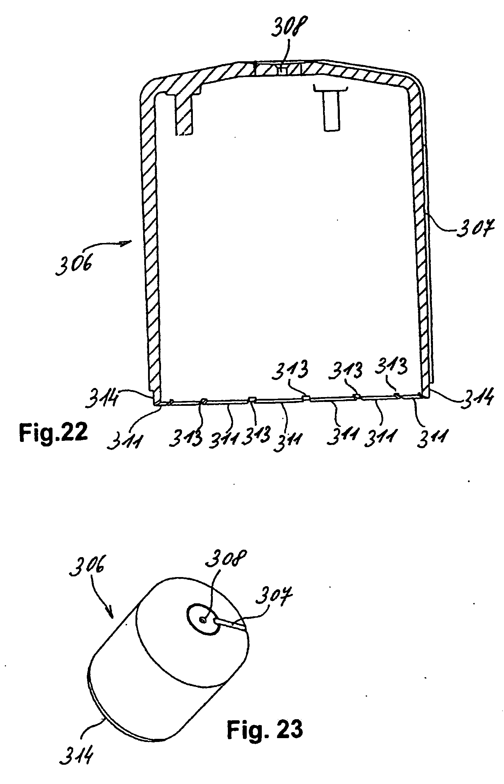

In the side walls of the second housing 306 -or alternatively in the walls of the outer upper housing 303- one or more connecting grooves 307 are made (Figures 22, 23), which put in fluid communication the reservoir 304 with the spray nozzle 308; the liquid 305 -for example a medicament- in the reservoir 304 is sucked from the reservoir 304 through the connecting groove -or connecting grooves- 307 by means of a Venturi effect obtained by injecting a pressurized gas flow through the gas intake 309 and the gas nozzle 310 into the orifice 308. The liquid 305 ejected from the spray nozzle 308 impacts against the atomization baffle 302 which brakes the liquid jet and some drops in smaller droplets so as to form a mist; the mist follows a radial flow path which is first oriented outward around the atomization baffle 302, and after turns radially inward toward the edge 301A of the outlet passageway 301 and then to the patient through the tubular body 5 (Figure 1); the liquid drops which are too large to be suspended in the mist flow fall, by force of gravity, onto the upper surface of the atomization baffle and from there to the liquid reservoir 304 or directly in the liquid reservoir 304.

This way the baffle and the tubular edge 301A work as a filter to control the size of the mist droplets.

According to the second aspect of the present invention, the edge 311 of the inner upper housing (or second housing) 306 abuts against the bottom 312 of the first housing 300 and -in the present embodiment- in the edge 311 a plurality of notches 313 are made, so that the liquid 305 can flow through them from the reservoir 304 to the connecting groove -or grooves- 307; preferably but not necessarily the liquid flowing out from the notches 313 is collected in an annular groove 314 set around the lower edge 314 of the inner upper container 306, and directed to the connecting grooves 307.

Alternatively the reservoir 304 can be set in fluid communication with the connecting groove 307 through one or more grooves carved in the bottom 312 of the first housing 300, and the edge 311 of the second housing can be provided with or without the notches 313.

The notches 313 -or alternatively corresponding grooves carved in the bottom 312 of the first housing- provide a flow passage with fixed dimensions and cross-section. and avoid for example that the edge 311 is set too high above the bottom 312 -in this case the connecting groove 307 sucks too much air from the reservoir 304- or that the edge 311 is set too low with respect to the bottom 312 -in this case the fluid communication between the reservoir 304 and the spray nozzle 308 is too throttled.

Preferably but not necessarily the height of the notches 313 is lower than about 2 mm, and more preferably is about 0,5-0,8 mm; a third preferred range of the notch height is about 0,2-0,5 mm: this way, when a small amount of medicine is left in the reservoir 304 the notches 313 are anyway submerged by the meniscus of the liquid medicine ―the surface tension and capillarity characteristics in the most of cases are quite similar to the ones of water- and the connecting groove 307 suck medicine properly; if the notches height would be greater than the meniscus height of the medicine, with small amounts of medicine in the reservoir -for example the thinnest liquid layer that can wet the bottom of the reservoir- the connecting groove 307 would suck mostly air since the cross-section of the notches would not be fully submerged by the medicine rests, mostly air would enter the notches 313 instead of liquid medicine and the mist flow of the nebulizer would be reduced or stopped completely. In case that the air will stop the liquid medicine from going through the groove 307, there will be medicine left in the reservoir 304 at the end of the treatment.

Preferably also the width of the notches 313 is small enough to make the water- or medicine drops adhere to the notch borders by means of surface tension forces: for example the notches 313 have circular, semicircular or squared cross-sections, and a width preferably not greater than 3 mm, and more preferably not greater than 1 mm.

In the non limiting embodiment shown in Figures 21-23, the notches 313 have a semicircular shape with a diameter of 0,2 mm.

The third aspect of the present invention will now be described.

In the nebulizer of Figure 21, the downwardly projecting aerosol flow outlet passageway is defined by a cylindrical duct delimited by an inner intake edge 301A; the inner intake edge 301A is the first part of the downwardly projecting aerosol flow outlet passageway that the aerosol flow licks while being discharged out of the nebulizer 1'.

The atomization baffle 302 has approximately the shape of a cupola-like shell defining a lower concavity 317 delimited by an edge 302A.

According to the third aspect of the present invention, the inner intake edge 301A of the downwardly projecting aerosol flow outlet passageway is substantially set inside the concavity 317 on the lower side of the baffle 302, that is -Figure 21- the height H1 of the inner intake edge 301A with respect to the nebulizer bottom is greater than the height H2 of the edge 302A of the concavity 317: this forces the aerosol flow to follow a discharge path with a more accentuated S-shape, since the aerosol flow is forced first along a downward direction, after along an upward direction and thirdly along a second downward direction; this double-elbow path allows a better separation of the smaller droplets from the bigger droplets than for example if H1 ≤ H2.

Advantageously the baffle 302 is fixed to an inner part, of the second housing 306, which is higher than the baffle surface upon which said flow of high velocity gas and drops are impinged: this allows the air and mist to flow freely and easily inside the nebulizer, with less undesired turbulences than for example if the baffle is fixed to legs protruding from a part of the second housing 306 substantially lower than the baffle surface upon which said flow of high velocity gas and drops are impinged.

It will be appreciated that the above descriptions are intended only to serve as examples, and that many other embodiments are possible within the spirit and the scope of the present invention.

Each modification and version within the meaning and the equivalence sphere of the claims is meant to be encompassed therewith.

## Claims

1. A pneumatic nebulizer comprising:
a) at least one downwardly projecting aerosol flow outlet passageway;
b) at least one inlet for introduction of a flow of high velocity gas;
c) at least one orifice through which said flow of high velocity gas passes thereby causing a venturi effect;
d) at least one source of liquid in fluid communication with said at least one orifice such that liquid is drawn to said orifice by said venturi effect, said liquid thereby forming into drops and flowing with said high velocity gas; and
e) at least one baffle upon which said flow of high velocity gas and drops are impinged so as to atomize said drops into yet smaller droplets thereby forming a mist, said baffle deployed such that a flow-path of said mist substantially circumscribes said baffle so as to reach said at least one downwardly projecting aerosol flow outlet passageway.

2. The nebulizer according to claim 1, wherein said source of liquid is a reservoir configured as a bottom portion of a volume within a housing of the nebulizer.

3. The nebulizer according to claim 1 or 2, wherein at least a top portion of said downwardly projecting aerosol outlet passageway extends upwardly into said volume.

4. The nebulizer according to claim 3, wherein said volume substantially circumscribes at least a portion of said downwardly projecting aerosol outlet passageway.

5. The nebulizer according to any one of claims 2, 3 or 4, further comprising at least one fluid passageway configured at a radial extremity of said volume through which said fluid communication is established.

6. The nebulizer according to claim 5, further including a fluid passageway insert deployed within said volume, said fluid passageway insert configured so as to substantially abut at least one housing surface defining said volume, said at least one fluid passageway configured as at least one surface groove configured in the surface of one of said fluid passageway insert and said housing surface.

7. The nebulizer according to any one of claims 2 to 6, wherein said volume is configured as a substantially vertical cylindrical volume such that said orifice is configured at a top of said cylindrical volume and said downwardly projecting aerosol outlet passageway is configured at a bottom of said cylindrical volume.

8. The nebulizer according to claim 6 or 7, wherein said fluid passageway insert is configured substantially as a cylinder having an open end and a closed end such that said closed end is deployed adjacent to said top of said cylindrical volume, and said closed end includes at least a portion of said orifice.

9. The nebulizer according to any one of claims 6, 7 or 8, wherein said at least one surface groove is implemented as a plurality of circumferentially spaced apart grooves in the surface of one of said fluid passageway insert and said housing surface.

10. The nebulizer according to any one of claims 5 to 9, wherein said baffle is supported substantially above said downwardly projecting aerosol outlet passageway so as to constitute a flow path obstacle around which said mist flows in order to reach said downwardly projecting aerosol outlet passageway.

11. The nebulizer according to claim 10, wherein said baffle is configured substantially as a disk having a diameter larger than a diameter of a top opening of said downwardly projecting aerosol outlet passageway, said baffle being supported above said downwardly projecting aerosol outlet passageway such that a extreme radial edge of said baffle extends beyond said top opening so as to be aligned with a portion of said reservoir.
